(19) Europäisches Patentamt
European Patent Office
Office européen des brevets

(11) **EP 3 763 811 B1**

(12) **EUROPEAN PATENT SPECIFICATION**

(45) Date of publication and mention
of the grant of the patent:
**05.03.2025 Bulletin 2025/10**

(21) Application number: **19382590.8**

(22) Date of filing: **12.07.2019**

(51) International Patent Classification (IPC):
*C12N 9/12* (2006.01)    *C12Q 1/6844* (2018.01)
*C07K 14/005* (2006.01)    *C12Q 1/70* (2006.01)
*C07K 14/15* (2006.01)

(52) Cooperative Patent Classification (CPC):
**C12N 9/1276; C07K 14/005; C12Q 1/701;**
**C12Y 207/07049;** C12N 2740/13022

(54) **REVERSE TRANSCRIPTASE AND USES THEREOF**

REVERSE TRANSKRIPTASE UND VERWENDUNGEN DAVON

TRANSCRIPTASE INVERSE ET LEURS UTILISATIONS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**13.01.2021 Bulletin 2021/02**

(73) Proprietor: **IRIGEN, S.L.U.**
**50001 Zaragoza (ES)**

(72) Inventor: **GENZOR ASÍN, Carlos**
**50001 Zaragoza (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**WO-A1-2018/189184**    **WO-A2-2013/060819**

• **DATABASE Geneseq [online] 13 December 2018**
**(2018-12-13), "Moloney murine leukemia virus**
**(MMLV) mutant RT #10.", XP002795368, retrieved**
**from EBI accession no. GSP:BFT20439 Database**
**accession no. BFT20439**

• **YUTA KATANO ET AL: "Generation of**
**thermostable Moloney murine leukemia virus**
**reverse transcriptase variants using site**
**saturation mutagenesis library and cell-free**
**protein expression system", BIOSCI. BIOTECH.**
**BIOCHEM., vol. 81, no. 12, 6 November 2017**
**(2017-11-06), pages 2339 - 2345, XP055491176,**
**ISSN: 0916-8451, DOI: 10.1080/**
**09168451.2017.1394790**

• **A. BARANAUSKAS ET AL: "Generation and**
**characterization of new highly thermostable and**
**processive M-MuLV reverse transcriptase**
**variants", PROTEIN ENGINEERING DESIGN AND**
**SELECTION, vol. 25, no. 10, 12 June 2012**
**(2012-06-12), pages 657 - 668, XP055071799,**
**ISSN: 1741-0126, DOI: 10.1093/protein/gzs034**

• **YASUKAWA K ET AL: "Increase in thermal**
**stability of Moloney murine leukaemia virus**
**reverse transcriptase by site-directed**
**mutagenesis", JOURNAL OF BIOTECHNOLOGY,**
**ELSEVIER, AMSTERDAM, NL, vol. 150, no. 3, 8**
**October 2010 (2010-10-08), pages 299 - 306,**
**XP027483996, ISSN: 0168-1656, [retrieved on**
**20101101], DOI: 10.1016/J.JBIOTEC.2010.09.961**

• **AREZI BAHRAM ET AL: "Novel mutations in**
**Moloney Murine Leukemia Virus reverse**
**transcriptase increase thermostability through**
**tighter binding to template-primer", NUCLEIC**
**ACIDS RESEARCH, OXFORD UNIVERSITY**
**PRESS, GB, vol. 37, no. 2, 4 December 2008**
**(2008-12-04), pages 473 - 481, XP002556110,**
**ISSN: 1362-4962, [retrieved on 20081204], DOI:**
**10.1093/NAR/GKN952**

- **K. ZHU ET AL: "Requirement for Integrase during Reverse Transcription of Human Immunodeficiency Virus Type 1 and the Effect of Cysteine Mutations of Integrase on Its Interactions with Reverse Transcriptase", JOURNAL OF VIROLOGY, vol. 78, no. 10, 27 April 2004 (2004-04-27), pages 5045 - 5055, XP055112886, ISSN: 0022-538X, DOI: 10.1128/ JVI.78.10.5045-5055.2004**
- **S W BLAIN ET AL: "Nuclease activities of Moloney murine leukemia virus reverse transcriptase. Mutants with altered substrate specificities", JOURNAL OF BIOLOGICAL CHEMISTRY, vol. 268, no. 31, 5 November 1993 (1993-11-05), pages 23585 - 23592, XP055491482, ISSN: 0021-9258**

Remarks:
   The complete document including Reference Table(s) and the Sequence Listing(s) can be downloaded from the EPO website

**Description**

**FIELD OF THE INVENTION**

The present invention pertains to the fields of molecular and cellular biology. Particularly, it refers to a Moloney Murine Leukemia Virus (M-MLV) reverse transcriptase (RT) having increased thermal stability as compared to wild type M-MLV, characterized in that it comprises at least the mutation alanine to cysteine at the amino acid position 154 (A154C) and the mutation aspartic acid to cysteine at the amino acid position 224 (D224C), wherein a disulphide bond is formed between the thiol groups of the two cysteine amino acids at the amino acid positions 154 and 224.

**STATE OF THE ART**

In examining the structure and physiology of an organism, tissue or cell, it is often desirable to determine its genetic content. The genetic framework of an organism is encoded in the double-stranded sequence of nucleotide bases in the deoxyribonucleic acid (DNA) which is contained in the somatic and germ cells of the organism. The genetic content of a particular segment of DNA, or genes, is typically manifested upon production of the protein which the gene encodes. In order to produce a protein, a complementary copy of one strand of the DNA double helix is produced by RNA polymerase enzymes, resulting in a specific sequence of ribonucleic acid (RNA). This particular type of RNA, since it contains the genetic message from the DNA for production of a protein, is called messenger RNA (mRNA).

Within a given cell, tissue or organism, there exist myriad mRNA species, each encoding a separate and specific protein. This fact provides a powerful tool to investigators interested in studying genetic expression in a tissue or cell. mRNA molecules may be isolated and further manipulated by various molecular biological techniques, thereby allowing the elucidation of the full functional genetic content of a cell, tissue or organism.

One common approach to the study of gene expression is the production of complementary DNA (cDNA) clones. In this technique, the mRNA molecules from an organism are isolated from an extract of the cells or tissues of the organism. This isolation often employs solid chromatography matrices, such as cellulose or agarose, to which oligomers of thymidine (T) have been complexed. Since the 3' termini on most eukaryotic mRNA molecules contain a string of adenosine (A) bases, and since A base pairs with T, the mRNA molecules can be rapidly purified from other molecules and substances in the tissue or cell extract. From these purified mRNA molecules, cDNA copies may be made using the enzyme RT, which results in the production of single-stranded cDNA molecules. This reaction is typically referred to as the first strand reaction. The single-stranded cDNAs may then be converted into a complete double-stranded DNA copy (i.e. a double-stranded cDNA) of the original mRNA (and thus of the original double-stranded DNA sequence, encoding this mRNA, contained in the genome of the organism) by the action of a DNA polymerase. The protein-specific double-stranded cDNAs can then be inserted into a plasmid or viral vector, which is then introduced into a host bacterial, yeast, animal or plant cell. The host cells are then grown in culture media, resulting in a population of host cells containing (or in many cases, expressing) the gene of interest.

This entire process, from isolation of mRNA from a source organism or tissue to insertion of the cDNA into a plasmid or vector to growth of host cell populations containing the isolated gene, is termed "cDNA cloning." The set of cDNAs prepared from a given source of mRNAs is called a "cDNA library." The cDNA clones in a cDNA library correspond to the genes transcribed in the source tissue. Analysis of a cDNA library can yield much information on the pattern of gene expression in the organism or tissue from which it was derived.

Moloney Murine Leukemia Virus (M-MLV) RT is one of the retroviral RT which has been studied in the past. It contains a single subunit of 78 kDa with RNA-dependent DNA polymerase and RNase H activity. This enzyme has been cloned and expressed in a fully active form in *E. coli*. Human Immunodeficiency Virus (HIV) RT is a heterodimer of p66 and p51 subunits in which the smaller subunit is derived from the larger by proteolytic cleavage. The p66 subunit has both a RNA-dependent DNA polymerase and an RNase H domain, while the p51 subunit has only a DNA polymerase domain. Active HIV p66/p51 RT has been cloned and expressed successfully in a number of expression hosts, including *E. Coli.* Within the HIV p66/p51 heterodimer, the 51-kD subunit is catalytically inactive, and the 66-kD subunit has both DNA polymerase and RNase H activity.

An important factor which influences the efficiency of reverse transcription is the ability of RNA to form secondary structures (loops) or the presence of highly stable GC zones. Such secondary structures can form, for example, when regions of RNA molecules have sufficient complementarity to hybridize and form double stranded RNA. Generally, the formation of RNA secondary structures can be reduced by raising the temperature of solutions which contain the RNA molecules. Thus, in many instances, it is necessary to reverse transcribe RNA at high temperatures above 37°C, even above 65°C and close to 100°C. However, art known RTs generally lose activity and stability when they are incubated at high temperatures much above 65°C.

The following documents should also be considered as relevant state of the art:

- Patent document WO 2013/060819 A2 discloses mutants of the M-MLV RT at different positions, including A 154 and D224. The substitutions are said to be defective in RT activity. It does not disclose a variant of the M-MLV RT with the two specific substitutions G154C and D224C. It does not disclose the effects of any substitution on thermal stability.
- Patent document WO 2018/189184 AI discloses mutants of the reverse transcriptase from M-MLV with increased thermal stability.
- Katano et al. (2017) disclose saturation mutagenesis of M-MLV RT to obtain thermostable variants where variants were constructed. It does not disclose substitutions A154C + D224C. [Katano, Yuta et al. "Generation of thermostable Moloney murine leukemia virus reverse transcriptase variants using site saturation mutagenesis library and cell-free protein expression system." Bioscience, biotechnology, and biochemistry vol. 81,12 (2017): 2339-2345. doi:10.1080/09168451.2017.1394790]
- Baranauskas et al. (2012) disclose mutagenesis of M-MLV RT to obtain thermostable variants. None of the mutations as to cysteine or to introduce disulfide bridges to stabilize the RT. [Baranauskas, Aurimas et al. "Generation and characterization of new highly thermostable and processive M-MuLV reverse transcriptase variants." Protein engineering, design & selection: PEDS vol. 25,10 (2012): 657-68. doi:10.1093/protein/gzs034]
- Yasukawa et al. (2010) disclose mutagenesis of M-MLV RT to increase thermal stability. No mutations to cysteine to produce disulfide bond stabilized RT were disclosed, even less A154C + D224C. [Yasukawa, Kiyoshi et al. "Increase in thermal stability of Moloney murine leukaemia virus reverse transcriptase by site-directed mutagenesis." Journal of biotechnology vol. 150,3 (2010): 299-306. doi:10.1016/j.jbiotec.2010.09.961]
- Arezi et al. (2009) disclose site-directed mutagenesis of M-MLV RT to increase thermal stability. No mutation to cysteine to produce disulfide bond stabilized RT were disclosed. [Arezi, Bahram, and Holly Hogrefe. "Novel mutations in Moloney Murine Leukemia Virus reverse transcriptase increase thermostability through tighter binding to template-primer." Nucleic acids research vol. 37,2 (2009): 473-81. doi: 10.1093/nar/gkn952]

The present invention is focused on solving the above cited problem and it refers to a M-MLV RT having increased thermal stability even at high temperatures above 65°C, as compared to wild type M-MLV.

## DESCRIPTION OF THE INVENTION

### Brief description of the invention

The present invention provides a RT enzyme, compositions comprising the same and methods useful in overcoming limitations of reverse transcription. In general, the present invention provides a modified or mutated RT such that the thermostability and/or fidelity of the enzyme are increased or enhanced. More particularly, the present invention is directed to the double M-MLV mutant A154C and D224C, wherein a disulphide bond is formed between the thiol groups of the two cysteine amino acids at the amino acid positions 154 and 224 (hereinafter the "RT of the invention" or "RTSS"). Surprisingly, the RT of the invention shows a high thermal stability, even at temperatures above 65°C to 100°C.

Particularly, such as it can be seen in the results provided in the present invention:

- The RT of the invention maintains its activity after incubation at high temperatures (preferably 65-80 °C), so it is especially appropriate to work with RNA comprising high secondary structure content and long transcripts (cDNA libraries) in which GC-rich templates can interfere in cDNA synthesis.
- The RT of the invention shows a low standard deviation and early Cqs (quantification cycle) when compared to other RTs. This confirms that the RT of the invention offers superior sensitivity and reproducibility to obtain reliable results.
- The RT of the invention loses less activity than the commercially available RT SuperScript™ IV Reverse Retrotranscriptase (Invitrogen, Thermofisher Scientific, Massachusetts, USA) when working at temperatures above 65 °C even with low concentration RNA samples.
- The RT of the invention has a thermostability advantage over SuperScript™ IV.

Consequently, the first embodiment of the present invention refers to a M-MLV RT having increased thermal stability as compared to wild type M-MLV reverse transcriptase, characterized in that it comprises the mutation alanine to cysteine at the amino acid position 154 of the SEQ ID NO: 1 (A154C) and the mutation aspartic acid to cysteine at the amino acid position 224 of the SEQ ID NO: 1 (D224C), wherein a disulphide bond is formed between the thiol groups of the two cysteine amino acids at the amino acid positions 154 and 224.

The second embodiment of the present invention refers to an isolated nucleic acid molecule or vector comprising a nucleotide sequence encoding the RT of the invention.

The third embodiment of the present invention refers to a recombinant host cell which comprises the above cited nucleic acid molecule or vector.

The fourth embodiment of the present invention refers to a composition comprising the RT of the invention.

The fifth embodiment of the present invention refers to a kit adapted for making, amplifying or sequencing nucleic acid molecules comprising a first container which in turn comprises at least the RT of the invention.

The sixth embodiment of the present invention refers to an *in vitro* method of reverse transcription to generate complementary DNA (cDNA) from a nucleic acid template molecule, comprising mixing the nucleic acid template with at least the RT of the invention, and incubating the mixture under conditions sufficient to make nucleic acid molecules complementary to all or a portion of the template.

The seventh embodiment of the present invention refers to an *in vitro* method for sequencing a nucleic acid molecule comprising, mixing the nucleic acid template to be sequenced with one or more primers, with at least the RT of the invention, one or more nucleotides and one or more terminating agents; incubating the mixture under conditions sufficient to synthesize a population of molecules complementary to all or a portion of the molecule being sequenced; and separating the population to determine the nucleotide sequence of all or a portion of the molecule being sequenced.

The eight embodiment of the present invention refers to an *in vitro* method for amplifying a nucleic acid template molecule, comprising mixing the nucleic acid template with at least the RT of the invention and one or more DNA polymerases, and incubating the mixture under conditions sufficient to amplify nucleic acid molecules complementary to all or a portion of the template.

The ninth embodiment of the present invention refers to the *in vitro* use of the RT of the invention to generate complementary DNA (cDNA) from a nucleic acid template molecule, for sequencing a nucleic acid molecule or for amplifying a nucleic acid template molecule.

In a preferred embodiment, the method for amplifying a nucleic acid template molecule comprises a polymerase chain reaction (PCR), preferably quantitative PCR (qPCR) or reverse transcription polymerase chain reaction (RT-PCR).

In a preferred embodiment, the methods of the invention are performed at a temperature above 65°C, preferably above 95°C.

In a preferred embodiment, the RT of the invention is characterized by the SEQ ID NO: 1, which comprises the residues 22-704 of wild type M-MLV RT with mutations A154C and D224C, and further comprises the N-terminal extension of sequence MGSSHHHHHHSSGLEVLFQGP:

**MGSSHHHHHHSSGLEVLFQGP**GSTWLSDFPQAWAETGGMGLAVRQAPLIIPLKAT
STPVSIKQYPMSQEARLGIKPHIQRLLDQGILVPCQSPWNTPLLPVKKPGTNDYRPVQ
DLREVNKRVEDIHPTVPNPYNLLSGLPPSHQWYTVLDLKD**C**FFCLRLHPTSQPLFAFE
WRDPEMGISGQLTWTRLPQGFKNSPTLFDEALHRDLADFRIQHPDLILLQYV**C**DLLL
AATSELDCQQGTRALLQTLGNLGYRASAKKAQICQKQVKYLGYLLKEGQRWLTEA
RKETVMGQPTPKTPRQLREFLGTAGFCRLWIPGFAEMAAPLYPLTKTGTLFNWGPD
QQKAYQEIKQALLTAPALGLPDLTKPFELFVDEKQGYAKGVLTQKLGPWRRPVAYL
SKKLDPVAAGWPPCLRMVAAIAVLTKDAGKLTMGQPLVILAPHAVEALVKQPPDR
WLSNARMTHYQALLLDTDRVQFGPVVALNPATLLPLPEEGLQHNCLDILAEAHGTR
PDLTDQPLPDADHTWYTDGSSLLQEGQRKAGAAVTTETEVIWAKALPAGTSAQRAE
LIALTQALKMAEGKKLNVYTDSRYAFATAHIHGEIYRRRGLLTSEGKEIKNKDEILAL
LKALFLPKRLSIIHCPGHQKGHSAEARGNRMADQAARKAAITETPDTSTLLIENSSPN
SRLIN

For the purpose of the present invention the following terms are defined:

- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of". Thus, the phrase "consisting of" indicates that the listed elements are required or mandatory, and that no other elements may be present.
- RTs assayed in the present invention:

    ◦ RTH- : M-MLV RT with the mutation D524N.
    ◦ RTSS: M-MLV RT of the invention with mutations A154C and D224C and disulphide bond formation.
    ◦ RTSSX: M-MLV RT with mutations A154C and D224C without disulphide bond.
    ◦ RTWT: M-MLV Wild Type RT.

∘ Tetro Reverse Transcriptase M-MLV: It is a commercially available RT which is commercialized by the company Bioline Meridian Bioscience, Ohio, USA.
∘ SuperScript™ IV: Commercially available RT which is commercialized by the company Invitrogen, Thermofisher Scientific, Massachusetts, USA.

## Brief description of the figures

**Figure 1.** Thermal denaturation of RTWT (dashed line), RTH- (discontinuous lines), RTSS (solid circles) and RTSSX (thin line). Fluorescence (arbitrary units) of enzymes at 5 $\mu$M was measured during a thermal ramp from 1 to 90 °C.

**Figure 2.** Loss of RTWT (grey bar) activity after incubation at 65°C. RTSS (black bar) was able to carry out retrotranscription and amplification through all conditions tested, even at highest temperatures. These experiments demonstrate that the thermostability is due to strategic mutations located in A154C and D224C in combination with disulphide bond formation.

**Figure 3.** RTSS (circles) displays up to 7 Cqs earlier than RTWT (squares). RTSS exhibits lowest standard deviation through different temperatures tested when compared to RTWT version. Dilution from a rotavirus positive sample (4607 $10^{-1}$) along different pre-incubation temperatures for both RTs is shown.

**Figure 4.** Amplification plot for a rotavirus positive sample (4607 $10^{-1}$) after Real Time RT PCR over a range of preincubation temperatures (66°C-82°C), using RTSS (circles) and RTWT (squares). The retrotranscription step was performed at 65°C during 15 min.

**Figure 5.** Loss of activity for SuperScript™ IV after incubation as compared with RTSS. Comparison with experiment w/o incubation. Dilution sample $10^{-2}$.

## Detailed description of the invention

## Example 1. Material and Methods.

### Example 1.1. Cloning.

DNA sequence for Recombinant Hyperstable RT (RTSS) was synthesized by GenScript (Hong-Kong), and codons were optimized for expression in *E. coli* cells. The recombinant sequence corresponding to residues 22-683 of the wild type Moloney Murine Leukemia Virus RT, with the mutations A154C and D224C, preferably fused to a His-Tag in the N-terminal and a 3C protease site for cleavage of His tag, was cloned in a vector derived from the p-ET15b plasmid. The final sequence expressed was the SEQ ID NO: 1.

### Example 1.2. Expression.

The plasmid was transformed in an *E. coli* BL21 DE3 strain and cultured at 30°C in LB medium until DO 0.6 and induced with 1mM Isopropyl β-D-1-thiogalactopyranoside (IPTG). After induction, culture temperature was lowered down to 18°C. After 12 hours, cells were harvested and cell pellet was stored frozen at -20 C.

### Example 1.3. Purification.

Pellet was resuspended in buffer 30 mM Hepes, 300 mM NaCl, 20 mM Imidazole, pH 7.4 and loaded in a His-Trap column (GE Healthcare). Elution was performed with buffer 30 mM Hepes, 300 mM NaCl, 500 mM Imidazole, pH 7.4 and the enzyme was dialyzed first against buffer 30 mM Hepes, 300 mM NaCl, 2mM cysteine, 1 mM cystine, pH 7.4 for 48 hours (for disulfide bond formation) and after that against buffer 30 mM Hepes, 300 mM NaCl, 2pH 7.4 at 4C. Finally, the enzyme was aliquoted, frozen in liquid nitrogen and stored at -80 C.

### Example 1.4. Thermal Stability.

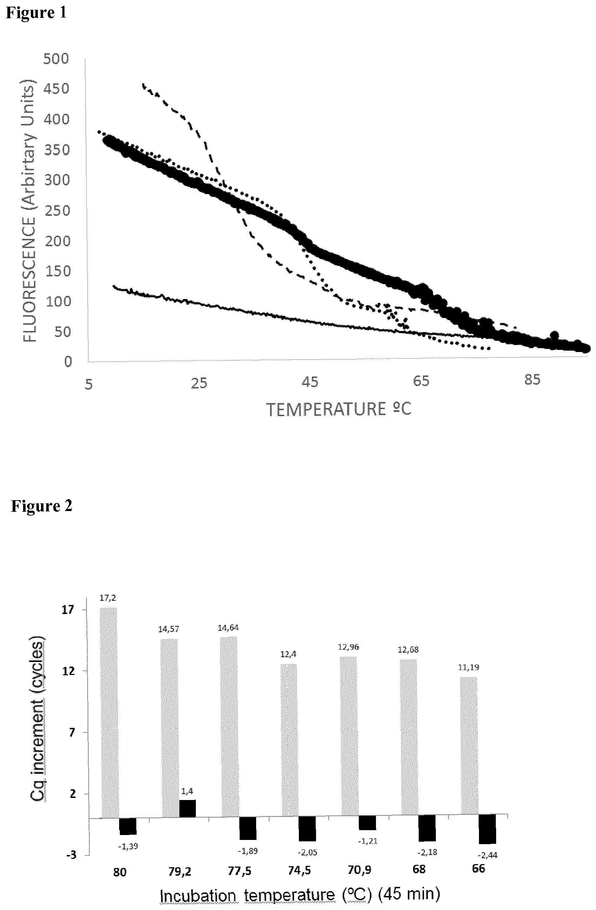

Thermal denaturation of RTSS was followed by tryptophan fluorescence emission. RTWT, and RTH- which is known to be more stable that the wild type version, were also analysed for comparison (**Figure 1**). To understand the role of disulphide bond formation, the RTSS enzyme without disulphide bond formation (RTSSX), dialyzed without cysteine and cysteine during purification, was also characterized (**Figure 1**).

Curves were recorded from 1 to 90 °C. Enzyme concentrations were 5 μM, excitation wavelength was 280 nm and fluorescence emission was recorded at 350 nM (tryptophan emission).

Unfolding curves were fitted to two or three-state equations depending on the case.

For the simplest model, a two-state equation where:

$$F = \frac{F_N + m_N T + (F_D + m_D T)e^{-\Delta G/RT}}{1 + e^{-\Delta G/RT}}$$

F is the spectroscopic signal, T corresponds to the absolute temperature, FN and FD, the spectroscopic signals of native and denatured enzymes at a temperature (T0) vary linearly with temperature with slopes mN and mD.

ΔG, free energy of the unfolding process, corresponds to:

$$\Delta G = \Delta H(1 - T/T_m) + \Delta C_p(T - T_m - T\ln(T/T_m))$$

Tm is the melting temperature (Temperature where 50 % of the molecules are in a folded state and 50 % in a denatured state). ΔH enthalpy change and ΔCp the heat capacity both considered at the melting temperature.

The melting temperature and the extension of the transition at a Temperature (expressed as a Fluorescence decrease) are the most powerful indicators of thermal stability.

The results obtained are shown in **Table 1.** In order to interpret the results, it is important to note that the greater the Tm, and the lower the loss of fluorescence in a transition, the greater the stability of a protein.

**Table 1**

|        | Tm1 (°C) | Tm2 (°C) |
|--------|----------|----------|
| RTWT   | 32       | -        |
| RTH-   | 43       | 62       |
| RTSS   | 45       | 70       |
| RTSSX  | -        | -        |

The unfolding for the enzyme RTWT is a two-step process with a Tm of 32 °C. The mutant RTH- is much more stable, with a first transition characterized by a Tm of 43 °C with and a second denaturation at 62 °C. The highest stability was provided by the RTSS comprising disulphide bond. RTSS is not only defined by their higher Tm1 and Tm2 (45 and 70 °C, respectively) but for the much smaller extent of the first transition (see **Figure 1** the small descending of fluorescence during this change of state). The most likely explanation for this fact is that the onset of unfolding that occurs locally at this temperature is restricted to a small portion of the protein, due to the disulphide bond presence. As it will be seen in the functional experiments performed by q-PCR, this higher thermal stability correlates with the ability of the RTSS enzyme to develop its retrotranscriptase activity at a higher temperature, compared for example with RTWT.

Consequently, the presence of two specific mutations A154C and D224C, along with the formation of the disulphide bond in those specific positions, give rise to an increase in the stability of the RT of the invention.

The thermal denaturation of the RTSSX, followed by emission of fluorescence, reveals that, already at very low temperatures, the RTSSX is in a partially unfolded state, as can be seen by the low fluorescence signal compared with the other enzymes. In addition, the enzyme with the unformed disulphide bond completely lacks detectable retrotranscriptase activity.

**Example 1.5. Stool samples and RNA extraction.**

RNA extraction of stool samples from patients with suspicious of rotavirus diarrhea was performed using INVISORB® Spin Universal kit (Stratec) following manufacture's protocol. 10-fold serial dilutions of RNA extracted were prepared in TE 1X low EDTA buffer. RNA extract dilutions were stored at -80°C until Real Time RT-PCR testing.

**Example 1.6. Functional assay by Real Time RT-PCR.**

Extracted RNA were used for one step Real Time PCR using different RT enzymes: RTH-, RTSS, RTSSX, RTWT, Tetro Reverse Transcriptase M-MLV and SuperScript™ IV Reverse Retrotranscriptase.

The Real RT-PCR assay was performed by using SensiFAST®Probe No-ROX Kit (Bioline meridian Bioscience). Each 20μl reaction mixture contained 10μl of 2x SensiFAST™ Probe No-ROX Mix, 250nM (each) forward and reverse primer, 125nM probe, nuclease-free water, 100Units of selected retrotranscriptase and 5μl of extracted RNA. Primers and probe were designed for specific Rotavirus A detection generating a multiple sequence alignment with MAFFT and confirming the specificity of the selected sequence using BLAST.

Custom primers and probes were ordered from IDT (Integrated DNA Technologies, Iowa, USA).

After adding to the reaction mixture the different RTs, the enzymes were pre incubated at different temperatures. Enzyme incubation, retrotranscription and amplification were carried out on a CFX96 Touch®Real-Time PCR Detection System (BioRad). Thermocycling conditions consisted of 15 min at 56°C-100°C for RT (depending on purpose experiment), polymerase activation at 95°C for 2 min; followed by up to 45 cycles of denaturation at 95°C for 10 seconds and annealing/extension at 60°C for 50 seconds.

Experiments of functional assays consist of small RNA fragments reverse transcription coupled with DNA amplification in which the different RTs were previously incubated at different temperatures. Pre incubation mimics conditions of working at high temperature with strong secondary RNA or full length cDNA synthesis. The purpose of this analysis is to check that RTSS was able to work at increased temperatures and to compare its functionality with other different RTs in terms of Ct displacement value and presence of amplification and therefore its ability to generate cDNA from very little input RNA.

We perform two types of experiments:

- Comparative studies of RTSS with different RTs variants (including RTWT) and other commercially available M-MLV Wild type RTs.
- Verification studies comparing RTSS vs. SuperScript™ IV. Please note that SuperScript™ IV is considered one of the most efficient and thermostable commercially available retrotranscriptase.

**Example 2. Results.**

On the first evaluation studies RTSS was compared with the RTSSX, RTH-, RTWT, and Tetro Reverse Transcriptase M-MLV. On the first experiment all RTs were pre incubated during 45 min at 60°C, 70°C or 80°C. The conditions for reverse transcription step were 45 minutes to different temperatures ranging from 56°C to 80°C. The results obtained were shown in **Table 2** and **Figure 1.** RTSS was able to carry out retrotranscription and amplification through all conditions tested, even at highest temperatures. These experiments demonstrate that the thermostability is due to strategic mutations located in A154C and D224C in combination with disulfide bond formation.

**Table 2.** Evaluation of ability to work at increased temperature at a range of pre incubation and reverse transcription temperatures for selected RTs.

**Table 2**

| Pre-incubation Temperature ( ºC) | Reverse Transcription Temperature (ºC) | Certest RTSS | Certest RTH- | Certest RTSSX | Certest M-MLV RTWT | Tetro RT (Bioline) |
|---|---|---|---|---|---|---|
| **80ºC** | **80** | 32,64 | Neg | Neg | Neg | 39,5 |
| | **78,6** | 35,35 | Neg | Neg | Neg | Neg |
| **70ºC** | **71,1** | 29,67 | Neg | Neg | 34,45 | Neg |
| | **65,3** | 29,16 | Neg | Neg | 34,43 | 34,07 |
| **60ºC** | **60,7** | 29,21 | Neg | Neg | 34,17 | 35,1 |
| | **57,6** | 29,42 | Neg | Neg | 34,68 | 36,42 |
| | **56ºC** | 29,51 | Neg | Neg | 34,43 | 35,92 |

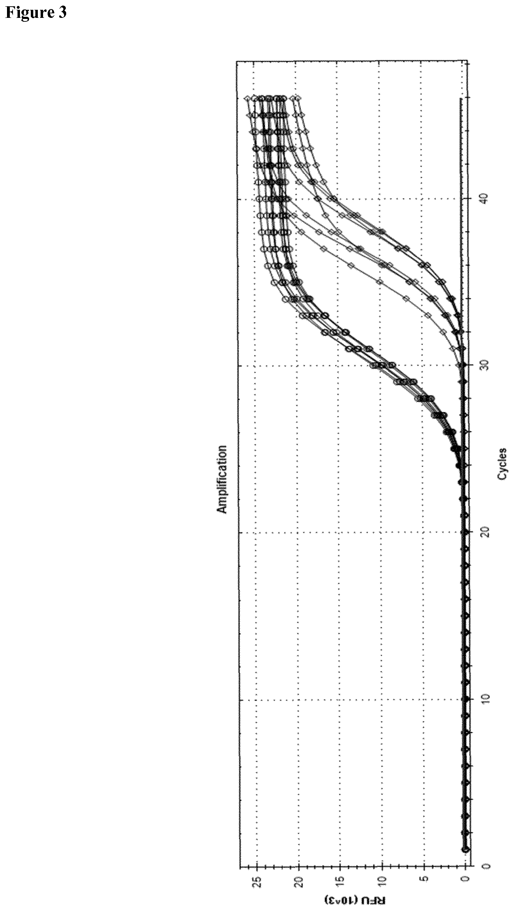

The results obtained with Real Time RT PCR from 10-fold dilutions of nucleic acid from positive rotavirus sample with RTSS and RTWT comparison at different incubations times is shown in **Table 3.** Corresponding amplification plot (**Figure 3**) shows that RTSS presents more reproducible results than RTWT over a range of high temperatures (65°C-80°C).

**Table 3.** RTSS sensitivity evaluation vs RTWT. The RTs were pre incubated during 45 minutes along a different range of high temperatures (65°C-80°C). The conditions of the retrotranscription step were 15 min, at 65°C.

EP 3 763 811 B1

Table 3

| Incubation Temperature (ºC) | Certest RTSS | | | | | MMLV RT-WT | | | | |
|---|---|---|---|---|---|---|---|---|---|---|
| | Sample dilution | | | | | Sample dilution | | | | |
| | 4607 $10^{-1}$ | 4607 $10^{-2}$ | 4607 $10^{-3}$ | 4607 $10^{-4}$ | 4607 $10^{-5}$ | 4607 $10^{-1}$ | 4607 $10^{-2}$ | 4607 $10^{-3}$ | 4607 $10^{-4}$ | 4607 $10^{-5}$ |
| 80 | 23,4 | 26,38 | 30,06 | 33,97 | 36,02 | 31,69 | 33,13 | 36,69 | Neg | Neg |
| 79,2 | 23,27 | 26,33 | 30,05 | 32,75 | 42,32 | 31,43 | 33,14 | 36,6 | Neg | Neg |
| 77,5 | 23,51 | 26,51 | 30,38 | 33,3 | 35,21 | 31,78 | 34,04 | 38,43 | Neg | Neg |
| 74,5 | 23,38 | 26,3 | 30,13 | 33,11 | 36,6 | 31,55 | 34,56 | 36,01 | Neg | Neg |
| 70,9 | 22,9 | 25,89 | 29,59 | 32,7 | 39,24 | 30,16 | 32,85 | 35,9 | Neg | Neg |
| 68 | 22,98 | 26,07 | 29,40, | 32,57 | 34,3 | 30,1 | 33,13 | 34,81 | 36,61 | 36,88 |
| 66 | 22,7 | 25,74 | 29,25 | 32,87 | 35,97 | 28,84 | 32,32 | 36,23 | 38,56 | Neg |
| 65 | 23,02 | 25,84 | 30,53 | 32,65 | 38,4 | 30,28 | 33,28 | 38,87 | 37,72 | Neg |
| Average | 23,14 | 26,13 | 29,92 | 32,99 | 37,25 | 30,72 | 33,3 | 36,69 | Neg | Neg |

Regarding verification studies comparing RTSS vs. SuperScript™ IV, the experiments performed were based on pre incubations of RTs at different point of temperatures during 45 minutes (**Table 4**). The results demonstrate that in the 66-80°C range, loss of activity is much more important for SuperScript™ IV. Above 65 °C, RTSS works as well as SuperScript™ IV with high concentration RNA samples and better with low concentration RNA samples.

**Table 4.** RTSS sensitivity evaluation comparing vs. SuperScript™ IV.

**Table 4**

| Incubation Temperature ($^{\circ}$C) | SuperScript IV | | | | Certest RTSS | | | |
|---|---|---|---|---|---|---|---|---|
| | Sample dilution | | | | Sample dilution | | | |
| | $10^{-1}$ | $10^{-2}$ | $10^{-3}$ | $10^{-4}$ | $10^{-1}$ | $10^{-2}$ | $10^{-3}$ | $10^{-4}$ |
| 79,2 | 24,9 | 27,89 | 31,88 | Neg | 24,01 | 27,36 | 30,45 | 32,8 |
| 77,5 | 25,19 | 28,73 | 31,37 | Neg | 24,72 | 27,56 | 32,22 | 35,8 |
| 74,5 | 24,06 | 27,79 | 32,04 | Neg | 26,84 | 31,39 (*) | 34,14 | Neg |
| 70,9 | 25,31 | 27,62 | 31,83 | Neg | 25,82 | 26,9 | 31,4 | 33,81 |
| 68 | 24,17 | 26,39 | 30,53 | Neg | 24,08 | 27,11 | 30 | 33,24 |
| 66 | 24,21 | 27,76 | 31,55 | Neg | 23,82 | 27,05 | 29,04 | 32,62 |
| **AVERAGE** | **24,6** | **27,6** | **31,5** | **Neg** | **24,8** | **27,1** | **31,2** | **33,6** |
| w/o incubation | 16,57 | 21,73 | 29,34 | Neg | 22,12 | 25,3 | 29,14 | 32,99 |

When RTSS was subjected to high pre incubation temperatures (81°C-100°C), it displays high stability (**Table 5**).

**Table 5.** Thermostability experiments with RTSS at very high temperatures. The time of preincubation is 45 minutes. Conditions of retrotranscription step were 15 minutes at 65°C.

**Table 5**

| Incubation Temperature ($^{\circ}$C) | Certest RTSS | |
|---|---|---|
| | Sample dilution | |
| | $10^{-2}$ | $10^{-3}$ |
| 100 | **30,8** | **33,15** |
| 99 | 32,83 | 35,73 |
| 96,7 | **30,97** | **34,29** |
| 92,7 | 29,88 | Neg* |
| 87,9 | 29,65 | 32,63 |
| 84,1 | 28,66 | 34,8 |
| 81,4 | 29,5 | 32,24 |

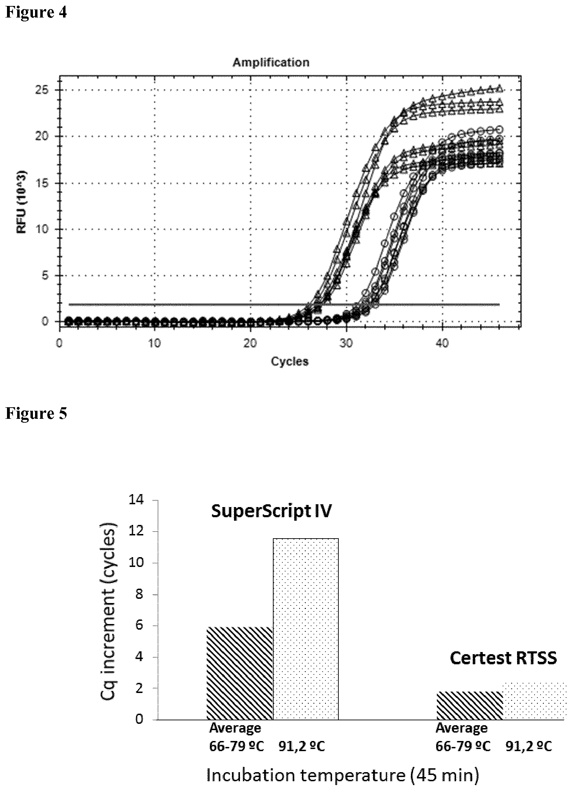

One of the high temperatures was chosen (91°C) and we performed the experiment using SuperScript™ IV as a reference thermostability enzyme. Loss of activitiy is much more evident in SuperScript™ IV than in RTSS at very high temperatures (**Table 6, Figure 5**).

**Table 6.** Very high temperature activity RTSS vs. SuperScript™ IV comparison.

**Table 6**

| Incubation Temperature ($^{\circ}$C) | SuperScript IV | | Certest RT | |
|---|---|---|---|---|
| | Sample dilution | | Sample dilution | |
| | 10-2 | 10-3 | 10-2 | 10-3 |
| 91,2 | 33,88 | 40,2 | 29,48 | 36,1 |

**Claims**

1.  A Moloney Murine Leukemia Virus (M-MLV) reverse transcriptase having increased thermal stability as compared to wild type M-MLV reverse transcriptase, **characterized in that** it comprises the mutation alanine to cysteine at the amino acid position 154 of the SEQ ID NO: 1 (A154C) and the mutation aspartic acid to cysteine at the amino acid position 224 of the SEQ ID NO: 1 (D224C), wherein a disulfide bond is formed between the thiol groups of the two cysteine amino acids at the amino acid positions 154 and 224.

2.  The Moloney Murine Leukemia Virus (M-MLV) reverse transcriptase, according to claims 1, **characterized by** the SEQ ID NO: 1.

3.  An isolated nucleic acid molecule or vector comprising a nucleotide sequence encoding the M-MLV reverse transcriptase of any of the previous claims.

4.  A recombinant host cell which comprises a nucleic acid molecule or vector according to claim 3.

5.  A composition comprising the M-MLV reverse transcriptase of any of claims 1 or 2.

6.  A kit adapted for making, amplifying or sequencing nucleic acid molecules comprising a first container which in turn comprises at least the M-MLV reverse transcriptase of any of the claims 1 or 2.

7.  *In vitro* method of reverse transcription to generate complementary DNA (cDNA) from a nucleic acid template molecule, comprising mixing the nucleic acid template with at least the M-MLV reverse transcriptase of any of the claims 1 or 2, and incubating the mixture under conditions sufficient to make nucleic acid molecules complementary to all or a portion of the template.

8.  *In vitro* method for sequencing a nucleic acid molecule comprising, mixing the nucleic acid template to be sequenced with one or more primers, with at least the M-MLV reverse transcriptase of any of the claims 1 or 2, one or more nucleotides and one or more terminating agents; incubating the mixture under conditions sufficient to synthesize a population of molecules complementary to all or a portion of the molecule being sequenced; and separating the population to determine the nucleotide sequence of all or a portion of the molecule being sequenced.

9.  *In vitro* method for amplifying a nucleic acid template molecule, comprising mixing the nucleic acid template with at least the M-MLV reverse transcriptase of any of the claims 1 or 2 and one or more DNA polymerases, and incubating the mixture under conditions sufficient to amplify nucleic acid molecules complementary to all or a portion of the template.

10. The method for amplifying a nucleic acid template molecule, according to claim 9, **characterized in that** it comprises a polymerase chain reaction (PCR), preferably quantitative PCR (qPCR) or reverse transcription polymerase chain reaction (RT-PCR).

11. The method, according to claim 7, **characterized in that** it is performed at a temperature above 65°C, preferably above 95°C.

12. *In vitro* use of the M-MLV reverse transcriptase of any of the claims 1 or 2 to generate complementary DNA (cDNA) from a nucleic acid template molecule, for sequencing a nucleic acid molecule or for amplifying a nucleic acid template molecule.

**Patentansprüche**

1.  Moloney-Maus-Leukämie-Virus (M-MLV)-Reverse Transkriptase aufweisend eine erhöhte Wärmestabilität im Vergleich zur Wildtyp-M-MLV-Reversen Transkriptase, **dadurch gekennzeichnet, dass** sie die Mutation Alanin zu Cystein an der Aminosäureposition 154 der SEQ ID NO: 1 (A154C) und die Mutation Asparaginsäure zu Cystein an der Aminosäureposition 224 der SEQ ID NO: 1 (D224C) umfasst, wobei eine Disulfidbindung zwischen den Thiol-Gruppen der beiden Cystein-Aminosäuren an den Aminosäurepositionen 154 und 224 gebildet wird.

2.  Moloney-Maus-Leukämie-Virus (M-MLV)-Reverse Transkriptase nach den Ansprüchen 1, **gekennzeichnet durch** die SEQ ID NO: 1.

3. Isoliertes Nukleinsäuremolekül oder Vektor umfassend eine Nukleotidsequenz, welche für die M-MLV-Reverse Transkriptase nach einem der vorhergehenden Ansprüche kodiert.

4. Rekombinante Wirtszelle, welche ein Nukleinsäuremolekül oder einen Vektor nach Anspruch 3 umfasst.

5. Zusammensetzung umfassend die M-MLV-Reverse Transkriptase nach einem der Ansprüche 1 oder 2.

6. Kit angepasst zur Herstellung, Amplifikation oder Sequenzierung von Nukleinsäuremolekülen umfassend einen ersten Behälter, welcher wiederum mindestens die M-MLV-Reverse Transkriptase nach einem der Ansprüche 1 oder 2 umfasst.

7. *In-vitro*-Verfahren der reversen Transkription, um komplementäre DNA (cDNA) aus einem Nukleinsäure-Templatmolekül zu erzeugen, umfassend das Mischen des Nukleinsäure-Templats mit mindestens der M-MLV-Reversen Transkriptase nach einem der Ansprüche 1 oder 2, und das Inkubieren der Mischung unter ausreichenden Bedingungen, um Nukleinsäuremoleküle komplementär zum gesamten oder zu einem Teil des Templats herzustellen.

8. *In-vitro*-Verfahren zur Sequenzierung eines Nukleinsäuremoleküls umfassend das Mischen des zu sequenzierenden Nukleinsäure-Templats mit einem oder mehreren Primern, mit mindestens der M-MLV-Reversen Transkriptase nach einem der Ansprüche 1 oder 2, einem oder mehreren Nukleotiden und einem oder mehreren Abbruchmitteln; das Inkubieren der Mischung unter ausreichenden Bedingungen, um einen Bestand von Molekülen komplementär zum gesamten oder zu einem Teil des zu sequenzierenden Moleküls zu synthetisieren; und das Trennen des Bestands, um die Nukleotidsequenz des gesamten oder eines Teils des zu sequenzierenden Moleküls zu bestimmen.

9. *In-vitro*-Verfahren zur Amplifikation eines Nukleinsäure-Templatmoleküls umfassend das Mischen des Nukleinsäure- Templats mit mindestens der M-MLV-Reversen Transkriptase nach einem der Ansprüche 1 oder 2 und einer oder mehreren DNA-Polymerasen, und das Inkubieren der Mischung unter ausreichenden Bedingungen, um Nukleinsäuremoleküle komplementär zum gesamten oder zu einem Teil des Templats zu amplifizieren.

10. Verfahren zur Amplifikation eines Nukleinsäure-Templatmoleküls nach Anspruch 9, **dadurch gekennzeichnet, dass** es eine Polymerase-Kettenreaktion (PCR), vorzugsweise eine quantitative PCR (qPCR) oder eine Reverse-Transkription-Polymerase-Kettenreaktion (RT-PCR) umfasst.

11. Verfahren nach Anspruch 7, **dadurch gekennzeichnet, dass** es bei einer Temperatur über 65 °C, vorzugsweise über 95 °C, durchgeführt wird.

12. *In-vitro*-Verwendung der M-MLV-Reversen Transkriptase nach einem der Ansprüche 1 oder 2, um komplementäre DNA (cDNA) aus einem Nukleinsäure-Templatmolekül zu erzeugen, um ein Nukleinsäuremolekül zu sequenzieren oder um ein Nukleinsäure-Templatmolekül zu amplifizieren.

**Revendications**

1. Transcriptase inverse de virus de la leucémie murine de Moloney (M-MLV) ayant une stabilité thermique accrue par rapport à la transcriptase inverse de M-MLV de type naturel, **caractérisée en ce qu'**elle comprend la mutation d'alanine en cystéine dans la position d'acide aminé 154 de SEQ ID NO : 1 (A154C) et la mutation d'acide aspartique en cystéine dans la position d'acide aminé 224 de SEQ ID NO : 1 (D224C), dans laquelle une liaison disulfure est formée entre les groupes thiol des deux acides aminés de cystéine dans les positions d'acide aminé 154 et 224.

2. Transcriptase inverse de virus de la leucémie murine de Moloney (M-MLV), selon les revendications 1, **caractérisée par** SEQ ID NO : 1.

3. Molécule d'acide nucléique isolée ou vecteur comprenant une séquence de nucléotides codant pour la transcriptase inverse de M-MLV selon l'une quelconque des revendications précédentes.

4. Cellule hôte recombinante qui comprend une molécule d'acide nucléique ou vecteur selon la revendication 3.

**5.** Composition comprenant la transcriptase inverse de M-MLV selon l'une quelconque des revendications 1 ou 2.

**6.** Kit adapté pour préparer, amplifier ou séquencer des molécules d'acide nucléique comprenant un premier récipient qui comprend à son tour au moins la transcriptase inverse de M-MLV selon l'une quelconque des revendications 1 ou 2.

**7.** Procédé *in vitro* de transcription inverse pour générer de l'ADN complémentaire (ADNc) à partir d'une molécule matrice d'acide nucléique, comprenant le mélange de la matrice d'acide nucléique avec au moins la transcriptase inverse de M-MLV selon l'une quelconque des revendications 1 ou 2, et l'incubation du mélange dans des conditions suffisantes pour produire des molécules d'acide nucléique complémentaires à la totalité ou une partie de la matrice.

**8.** Procédé *in vitro* pour séquencer une molécule d'acide nucléique comprenant le mélange de la matrice d'acide nucléique à séquencer avec un ou plusieurs amorces, avec au moins la transcriptase inverse de M-MLV selon l'une quelconque des revendications 1 ou 2, un ou plusieurs nucléotides et un ou plusieurs agents de terminaison ; l'incubation du mélange dans des conditions suffisantes pour synthétiser une population de molécules complémentaires à la totalité ou une partie de la molécule étant séquencée ; et la séparation de la population pour déterminer la séquence de nucléotides de la totalité ou une partie de la molécule étant séquencée.

**9.** Procédé *in vitro* pour amplifier une molécule matrice d'acide nucléique, comprenant le mélange de la matrice d'acide nucléique avec au moins la transcriptase inverse de M-MLV selon l'une quelconque des revendications 1 ou 2 et un ou plusieurs ADN polymérases, et l'incubation du mélange dans des conditions suffisantes pour amplifier des molécules d'acide nucléique complémentaires à la totalité ou une partie de la matrice.

**10.** Procédé pour amplifier une molécule matrice d'acide nucléique, selon la revendication 9, **caractérisé en ce qu'**il comprend une réaction en chaîne de la polymérase (PCR), de préférence PCR quantitative (qPCR) ou réaction en chaîne de la polymérase par transcription inverse (RT-PCR).

**11.** Procédé, selon la revendication 7, **caractérisé en ce qu'**il est réalisé à une température supérieure à 65 °C, de préférence supérieure à 95 °C.

**12.** Utilisation *in vitro* de la transcriptase inverse de M-MLV selon l'une quelconque des revendications 1 ou 2 pour générer de l'ADN complémentaire (ADNc) à partir d'une molécule matrice d'acide nucléique, pour séquencer une molécule d'acide nucléique ou pour amplifier une molécule matrice d'acide nucléique.

**Figure 1**

**Figure 2**

Figure 3

**Figure 4**

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013060819 A2

- WO 2018189184 A

### Non-patent literature cited in the description

- **KATANO, YUTA et al.** Generation of thermostable Moloney murine leukemia virus reverse transcriptase variants using site saturation mutagenesis library and cell-free protein expression system.. *Bioscience, biotechnology, and biochemistry*, 2017, vol. 81 (12), 2339-2345
- **BARANAUSKAS, AURIMAS et al.** Generation and characterization of new highly thermostable and processive M-MuLV reverse transcriptase variants. *Protein engineering, design & selection: PEDS*, 2012, vol. 25 (10), 657-68

- **YASUKAWA, KIYOSHI et al.** Increase in thermal stability of Moloney murine leukaemia virus reverse transcriptase by site-directed mutagenesis.. *Journal of biotechnology*, 2010, vol. 150 (3), 299-306
- **AREZI, BAHRAM ; HOLLY HOGREFE**. Novel mutations in Moloney Murine Leukemia Virus reverse transcriptase increase thermostability through tighter binding to template-primer.. *Nucleic acids research*, 2009, vol. 37 (2), 473-81